# EUROPÄISCHE PATENTSCHRIFT

(11) **EP 2 234 959 B1**
(45) Veröffentlichungstag und Bekanntmachung des Hinweises auf die Patenterteilung: **08.10.2014**
(21) Anmeldenummer: 08865830.7
(22) Anmeldetag: 10.12.2008
(51) Int. Cl.: C07C 209/26, C07C 209/62, C07C 211/07, C07C 211/27

(54) **EINSTUFIGE REDUKTIVE AMINIERUNG**
SINGLE-STEP REDUCTIVE AMINATION
AMINATION RÉDUCTRICE À UNE ÉTAPE

(30) Priorität: 21.12.2007 EP 07150298
(43) Veröffentlichungstag der Anmeldung: 06.10.2010
(73) Patentinhaber: BASF SE, 67056 Ludwigshafen (DE)
(72) Erfinder: STÄB, Tobias, 67227 Frankenthal (DE); HAHN, Thilo, 67292 Kirchheimbolanden (DE); SIEGEL, Wolfgang, 67117 Limburgerhof (DE)
(86) Internationale Anmeldenummer: PCT/EP2008/067192
(87) Internationale Veröffentlichungsnummer: WO 2009/080512

(56) Entgegenhaltungen:
- WO-A-2006/030017

## Beschreibung

Die Erfindung betrifft ein Verfahren zur Herstellung von Aminen der Formel I, 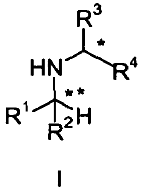 durch Umsetzung einer Carbonylverbindung der Formel II mit einem Amin der Formel III 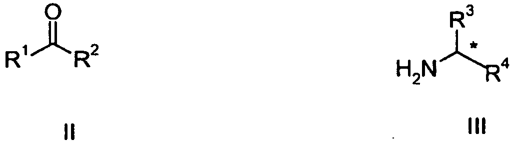 wobei
R¹ und R² voneinander verschieden sind und jeweils für einen organischen Rest mit 1 bis 20 C-Atomen, der gegebenenfalls auch Heteroatome enthalten kann, stehen,
R³ für eine C₁-C₆-Alkylgruppe steht und
R⁴ für eine Arylgruppe steht, welche partiell oder vollständig halogeniert sein kann und/oder ein bis drei Reste aus der Gruppe Cyano, Nitro, C₁-C₆-Alkyl; C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;
   und
   * für die S oder R-Konfiguration, und
   ** für die S und/oder R-Konfiguration;
   stehen;
   welches dadurch gekennzeichnet ist, dass man die Umsetzung in Gegenwart eines heterogen Iminierungs-Katalysators, eines Hydrierkatalysators und Wasserstoff durchführt, wobei es sich bei dem heterogenen Iminierungskatalysator um anorganische Oxide oder Mischoxide handelt.

Amine sind bedeutsame Synthesebausteine für Wirkstoffe auf dem Gebiet der Pharmazie und des Pflanzenschutzes. Bei chiralen Aminen (bei denen der Substituent am Amino-Stickstoff die Chiralität trägt) ist häufig nur eine stereoisomere Form (R oder S-Form), bzw. bei zwei Chiralitätszentren nur eine der diastereoisomeren Formen (RR, RS, SR, SS) geeignet.

Bei der Herstellung und Umsetzung von Aminen ist daher eine hohe Stereoselektivität bzw. Diastereoselektivität gewünscht.

Die Umsetzung von (prochiralen) Ketonen mit chiralen Aminen zu Aminoverbindungen, welche zwei Chiralitätszentren enthalten ist z. B. in EP-A-443 606 beschrieben. Nach der Lehre der EP-A 1640 358 und WO 2006/008171 wird diese Umsetzung in Gegenwart eines Hydrierkatalysatores und einer Lewissäure durchgeführt. Ein Nachteil dieser Synthese ist der überstöchiometrische und damit unwirtschaftliche Einsatz einer Lewissäure, die nach der Reaktion verworfen werden muss.

Aufgabe der vorliegenden Erfindung war daher ein möglichst einfaches und preisgünstiges Verfahren zur Herstellung von Aminoverbindungen mit zwei Chiralitätszentren durch Umsetzung von prochiralen Ketonen mit chiralen Aminen, wobei das erhaltenen Amin ein möglichst hohe Stereoselektivität haben soll, das Reaktionsprodukt leicht aufgearbeitet und gereinigt werden und der Katalysator möglichst einfach recycelbar ist.

Demgemäß wurde das eingangs definierte Verfahren gefunden.

### Zu den Edukten

Bei dem erfindungsgemäßen Verfahren wird eine Carbonylverbindung der Formel II mit einem Amin der Formel III umgesetzt.

Reste R1 und R2 in Formel II (und entsprechend natürlich auch im Produkt der Formel I) stehen jeweils unabhängig voneinander für einen organischen Rest mit 1 bis 20 C-Atomen, vorzugsweise mit 1 bis 10 C-Atomen, der gegebenenfalls auch Heteroatome enthalten kann. In einer bevorzugten Ausführungsform enthalten R1 und R2 keine Heteroatome und stehen für einen Kohlenwasserstoffrest, insbesondere einen aliphatischen Kohlenwasserstoffrest, ganz besonders bevorzugt für eine Alkylgruppe.

Als Reste R1 und R2 genannt seien z. B. :
C₁-C₆-Alkyl, C₃-C₆-Cycloalkyl, C₂-C₆-Alkenyl, C₂-C₆-Alkinyl, C₂-C₆-Halogenalkenyl, C₂-C₆-Halogenalkinyl, C₁-C₆-Alkoxycarbonyl, C₃-C₆-Alkenyloxycarbonyl, C₃-C₆-Alkinyloxy-carbonyl, Aminocarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, Di(C₁-C₆-alkyl)aminocarbonyl, N-(C₃-C₆-Alkenyl)-N-(C₁-C₆-alkyl)aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)aminocarbonyl, N-(C₃-C₆-Alkinyl)-N-(C₁-C₆-alkoxy)-aminocarbonyl, (C₁-C₆-Alkyl)aminothiocarbonyl, Di(C₁-C₆-alkyl)aminothio-carbonyl oder C₁-C₆-Alkylcarbonyl-C₁-C₆-alkyl,
   wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl-carbonylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
   Aryl, Aryl-C₁-C₄-alkyl, Aryl-C₂-C₄-alkenyl, Aryl-C₂-C₄-alkinyl, Aryl-C₁-C₄-halogenalkyl, Aryl-C₂-C₄-halogenalkenyl, Aryl-C₃-C₄-halogenalkinyl, Aryl-C₁-C₄-hydroxyalkyl, Arylcarbonyloxy-C₁-C₄-alkyl, Aryloxycarbonyl-C₁-C₄-alkyl, Aryloxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl, Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogenalkenyl, Heterocyclyl-C₃-C₄-halogenalkinyl, Heterocyclyl-C₁-C₄-hydroxyalkyl, Heterocyclylcarbonyloxy-C₁-C₄-alkyl, Heterocycloxycarbonyl-C₁-C₄-alkyl, Heterocyclyloxy-C₁-C₄-alkyl, Heterocyclylamino-C₁-C₄-alkyl Heterocyclylthio-C₁-C₄-alkyl, Heterocyclylsulfinyl-C₁-C₄-alkyl, Heterocyclylsulfonyl-C₁-C₄-alkyl,
   wobei die vorstehend genannten Aryl- und Heterocyclylreste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, Hydroxycarbonyl-C₁-C₆-alkyl, C₁-C₆-Alkoxycarbonyl-C₁-C₆-alkyl, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)aminocarbonylamino, Di(C₁-C₆-alkyl)aminocarbonylamino, Aryl und Aryl(C₁-C₆-alkyl) tragen können.

Die Reste R¹ und R² sind voneinander verschieden.

In Formel III (und entsprechend natürlich auch im Produkt der Formel I) haben die Reste R3 und R4 folgende Bedeutung:
R³ steht für eine C₁-C₆-Alkylgruppe steht und
R⁴ für eine Arylgruppe, welche partiell oder vollständig halogeniert sein kann und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;

Vorzugsweise steht R³ für eine Methylgruppe.

Vorzugsweise steht R⁴ für eine Phenyl- oder 1-Naphthylgruppe.
* in Formel I oder III bedeutet, dass die Verbindung entweder in der S-Konfiguration oder alternativ in der oder R-Konfiguration vorliegt. Es handelt sich daher nicht um ein Isomerengemisch, sondern eine stereospezifische Konfiguration
** in Formel steht für die S und/oder R-Konfiguration; d.h. die Verbindung kann an dieser Stelle anteilmäßig in beiden Konfigurationen vorliegen.

Die Carbonylverbindung II und das Amin III können bei der Umsetzung in beliebigen Mengen eingesetzt werden. Wenn eine Komponente im Überschuss eingesetzt wird, besteht die Notwendigkeit die nicht umgesetzten Mengen später aus dem Reaktionsgemisch abzutrennen; für eine optimale Reaktionsführung und/oder aus Kostengründen kann es aber vorteilhaft sein, eine der beiden Komponenten im Überschuss einzusetzen.

### Zu den Katalysatoren

Die Umsetzung wird in Gegenwart eines heterogenen Iminierungskatalysators durchgeführt, wobei es sich bei dem heterogenen Iminierungskatalysator um anorganische Oxide oder Mischoxide handelt. Solche Iminierungskatalysatoren katalysieren die Umsetzung von Carbonylverbindungen zu den entsprechenden Iminoverbindungen.

Wesentliches Merkmal der Erfindung ist, dass der Iminierungskatalysator heterogen ist, d.h. der Iminierungskatalysator ist unter den Reaktionsbedingungen ein Feststoff und liegt somit in einer anderen Phase vor als die Ausgangsstoffe der Umsetzung. Im Gegensatz dazu sind homogene Iminierungskatalysatoren, wie sie EP-A 1640358 oder WO 2006/008171 aufgeführt sind, in den Ausgangsstoffen oder gemeinsam mit den Ausgangsstoffen in einem Lösemittel gelöst, d.h. sie liegen in der gleichen Phase wie die Ausgangsstoffe vor (homogene Katalysatoren).

Vorzugsweise handelt es sich bei dem heterogenen Iminierungskatalysator um Feststoffe mit einem Schmelzpunkt größer 200 °C (1 bar). Der Iminierungskatalysator liegt vorzugsweise als Pulver oder körniges Material vor.

Als Oxide genannt seien z. B. TiO₂ oder ZrO₂.

Insbesondere handelt es sich um Silikate oder Silizium enthaltende Mischoxide.

Besonders geeignet sind Silikate, insbesondere Silikate, welche weitere Metallatome, wie Aluminium, Calcium Magnesium, Natrium, Kalium enthalten.

Genannt sei beispielsweise Montmorillonit. Montmorillonit hat im allgemeinen die Zusammensetzung (Na, Ca)_{0,3} (Al, Mg)₂ Si₄ (OH)₂ 4 H₂O.

Der Iminierungskatalysator wird vorzugsweise in Mengen von 0,1 bis 20 Gew. Teilen, besonders bevorzugt von 1 bis 15 Gew. Teilen und ganz besonders bevorzugt in Mengen von 2 bis 10 Gew. Teilen auf 100 Gew. Teile der Gewichtsumme der Ausgangsverbindungen II und III eingesetzt.

Die Umsetzung erfolgt weiterhin in Gegenwart eines Hydrierkatalysators.

Als Hydrierkatalysatoren sind alle Katalysatoren geeignet, welche für Hydrierungen verwendet werden.

Als aktiver Katalysatorbestandteil In Betracht kommen insbesondere Metalle oder Metalloxide. Genannt seien Edelmetalle, wie Platin, Palladium, Rhodium, Ruthenium und sonstige Metalle wie Nickel, Kobalt, Kupfer, Zink.

Geeignete Oxide sind insbesondere Nickel-, Kobalt-, Kupfer-, Zink-Oxide.

Der Hydrierkatalysator kann allein aus dem aktiven Katalysatorbestandteil bestehen, in einer bevorzugten Ausführungsform ist der aktive Katalysatorbestandteil auf einen inerten Träger aufgebracht (Trägerkatalysator). Bei dem Träger kann es sich um die übliche Träger wie Aluminiumoxid, Siliciumoxid, Titandioxid oder Calciumcarbonat handeln.

Bevorzugte Hydrierkatalysator sind wie die Iminierungskatalysatoren ebenfalls heterogene Katalysatoren, besonders bevorzugt sind Trägerkatalysatoren.

Besonders bevorzugt sind heterogene, Kupfer-haltige Katalysatoren, insbesondere Kupfer-haltige Trägerkatalysatoren als Hydrierkatalysatoren.

Die erfindungsgemäße Umsetzung wird daher vorzugsweise in Gegenwart eines heterogenen, Kupfer-haltigen Katalysators durchgeführt.

Vorzugsweise enthält dieser heterogene Kupfer-haltige Katalysator, bezogen auf das Gesamtgewicht des Katalysators
- 0,1 - 95 Gew. %: Kupfer;
- 0,1 - 85 Gew. %: mindestens ein Metall ausgewählt aus der Gruppe Nickel, Kobalt und Zink;
- 0 - 5% Gew. %: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur;
wobei die Summe der Gew. % 100% nicht übersteigt.

In der Regel enthält der heterogene Kupferhaltige Katalysator ein Trägermaterial. Als Trägermaterial kommt Kohle, z. B. Aktivkohle, Graphit oder Ruß, oder ein poröses Metalloxid in Betracht. Beispiele für geeignete poröse Metalloxide sind Aluminiumoxid, Siliciumdioxid, Alumosilicate, Titandioxid, Zirkoniumdioxid, Magnesiumoxid oder Gemische hiervon, vorzugsweise Aluminiumoxid, Titandioxid oder Zirkoniumdioxid. Es ist aber auch möglich als Trägermaterialien Aluminiumphosphate, Mullitte, Kieseiguhr, Bauxite und Caliumaluminate einzusetzen.

Insbesondere beträgt das Gesamtgewicht der oben genannten katalytisch aktiven Metalle und ggf. Promotoren des heterogenen Kupfer-haltigen Katalysators bezogen auf sein Gesamtgewicht höchstens 95 Gew.%, vorzugsweise höchstens 90 Gew.%.

In einer Ausgestaltungsform enthält dieser heterogene Kupfer-haltige Katalysator, bezogen auf das Gesamtgewicht des Katalysators
- 1 - 90 Gew.%: Kupfer;
- 0,1 - 80 Gew.%: mindestens ein Metall ausgewählt aus der Gruppe Nickel, Kobalt und Zink;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Insbesondere enthält dieser heterogene Kupfer-haltige Katalysator, bezogen auf das Gesamtgewicht des Katalysators
- 2 - 85 Gew.%: Kupfer;
- 0,1 - 80 Gew.%: mindestens ein Metall ausgewählt aus der Gruppe Nickel, Kobalt und Zink;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

In einer weiteren Ausgestaltungsform enthält dieser heterogene Kupfer-haltige Katalysator, bezogen auf das Gesamtgewicht des Katalysators
- 2 - 50 Gew.%: Kupfer;
- 0 - 30 Gew.%: mindestens ein Metall ausgewählt aus der Gruppe Nickel und Kobalt;
- 0,5 - 50 Gew. %: Zink;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Vorzugsweise enthält dieser heterogene Kupfer-haltige Katalysator, bezogen auf das Gesamtgewicht des Katalysators
- 5 - 40 Gew.%: Kupfer;
- 0 - 30 Gew.%: mindestens ein Metall ausgewählt aus der Gruppe Nickel und Kobalt;
- 5 - 50 Gew. %: Zink;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Insbesondere enthält dieser heterogene Kupfer-haltige Katalysator, bezogen auf das Gesamtgewicht des Katalysators
- 10 - 35 Gew.%: Kupfer;
- 0 - 30 Gew.%: mindestens ein Metall ausgewählt aus der Gruppe Nickel und Kobalt;
- 10 - 45 Gew. %: Zink;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Besonders bevorzugt enthält dieser heterogene Kupfer-haltige Katalysator, bezogen auf das Gesamtgewicht des Katalysators
- 10 - 35 Gew. %: Kupfer;
- 10 - 40 Gew. %: Zink;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Insbesondere bevorzugt enthält dieser Kupfer-haltige Katalysator, als katalytisch aktive Metalle nur Kupfer und Zink, insbesondere jeweils (aber unabhängig voneinander) 5 bis 50 Gew.%, besonders bevorzugt 10 bis 45 Gew.%, insbesondes bevorzugt 20 bis 40 Gew. %, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Katalysator, bezogen auf das Gesamtgewicht des Katalysators
- 2 - 50 Gew.%: Kupfer;
- 0,1 - 70 Gew.%: Nickel
- 0 - 20 Gew.%: mindestens eines Metalles ausgewählt aus der Gruppe Kobalt und Zink;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Vorzugsweise enthält dieser heterogene Kupfer-haltige Katalysator, bezogen auf das Gesamtgewicht des Katalysators
- 2 - 40 Gew.%: Kupfer;
- 1 - 65 Gew.%: Nickel;
- 0 - 10 Gew.%: mindestens eines Metalles ausgewählt aus der Gruppe Kobalt und Zink;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Insbesondere enthält dieser heterogene Kupfer-haltige Katalysator, bezogen auf das Gesamtgewicht des Katalysators
- 2 - 25 Gew.%: Kupfer;
- 3 - 60 Gew.%: Nickel;
- 0 - 10 Gew.%: mindestens ein Metall ausgewählt aus der Gruppe Gruppe Kobalt und Zink;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Besonders bevorzugt enthält dieser heterogene Kupfer-haltige Katalysator, bezogen auf das Gesamtgewicht des Katalysators
- 2 - 25 Gew.%: Kupfer;
- 3 - 50 Gew. %: Nickel;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur, vorzugsweise Molybdän.

Vorzugsweise enthält dieser Kupfer-haltige Katalysator, als katalytisch aktive Metalle nur Kupfer und Nickel, insbesondere jeweils (aber unabhängig voneinander) 2 bis 15 Gew.%, besonders bevorzugt 2 bis 10 Gew.%, insbesondes bevorzugt 3 bis 8 Gew.%, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht. (He: Beispiel mit jeweils 5 Gew% auf TiO₂)

Ebenso vorzugsweise enthält dieser Kupfer-haltige Katalysator, als katalytisch aktive Metalle bzw. Promotoren nur Kupfer, Nickel und Molybdän, insbesondere 2 bis 25 Gew.% Cu, 20 bis 60 Gew.% Nickel, 0,01 bis 5 Gew.% Molybdän, besonders bevorzugt 5 bis 20 Gew.% Kupfer, 30 bis 50 Gew.% Nickel, 0,1 bis 2 Gew.% Molybdän, insbesondes bevorzugt 10 bis 15 Gew.% Kupfer, 35 bis 45 Gew.% Nickel, 0,5 bis 1,5 Gew.% Molybdän bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Katalysator, bezogen auf das Gesamtgewicht des Katalysators
- 2 - 40 Gew.%: Kupfer;
- 0,1 - 80 Gew.%: mindestens ein Metall ausgewählt aus der Gruppe Nickel und Kobalt;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Vorzugsweise enthält dieser heterogene Kupfer-haltige Katalysator, bezogen auf das Gesamtgewicht des Katalysators
- 2 - 40 Gew.%: Kupfer;
- 0,1 - 40 Gew.%: Nickel;
- 0,1 - 40 Gew.%: Kobalt;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Insbesondere enthält dieser heterogene Kupfer-haltige Katalysator, bezogen auf das Gesamtgewicht des Katalysators
- 2 - 30 Gew.%: Kupfer;
- 0,5 - 35 Gew.%: Nickel;
- 0,5 - 35 Gew.%: Kobalt;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Besonders bevorzugt enthält dieser heterogene Kupfer-haltige Katalysator, bezogen auf das Gesamtgewicht des Katalysators
- 2 - 20 Gew.%: Kupfer;
- 1 - 30 Gew. %: Nickel;
- 1 - 30 Gew. %: Kobalt;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur, vorzugsweise Molybdän.

Vorzugsweise enthält dieser Kupfer-haltige Katalysator, als katalytisch aktive Metalle nur Kupfer, Nickel und Kobalt, insbesondere 2 bis 25 Gew.% Kupfer und jeweils unabhängig voneinander 1 bis 35 Gew.% Nickel bzw. Kobalt, besonders bevorzugt 2 bis 20 Gew.% Kupfer und jeweils unabhängig voneinander 10 bis 30 Gew.% Nickel bzw. Kobalt, insbesondes bevorzugt 5 bis 15 Gew. %Gew.% Kupfer und jeweils unabhängig voneinander 15 bis 25 Gew.% Nickel bzw. Kobalt, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht. (He: Beispiel mit 11/21/21Gew% auf TiO₂ bzw. ZrO2)

Ebenso insbesondere enthält dieser Kupfer-haltige Katalysator, als katalytisch aktive Metalle nur Kupfer, Nickel und Kobalt, besonders bevorzugt 2 bis 10 Gew.% Kupfer und jeweils unabhängig voneinander 1 bis 10 Gew.% Nickel bzw. Kobalt, insbesondere bevorzugt 2 bis 5 Gew.% Kupfer und jeweils unabhängig voneinander 2 bis 5 Gew.% Nickel bzw. Kobalt, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Katalysator, bezogen auf das Gesamtgewicht des Katalysators
- 2 - 40 Gew.%: Kupfer;
- 0,1 - 80 Gew.%: Kobalt;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Insbesondere enthält dieser heterogene Kupfer-haltige Katalysator, bezogen auf das Gesamtgewicht des Katalysators
- 2 - 20 Gew.%: Kupfer;
- 2 - 20 Gew.%: Kobalt;
- 0 - 5% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Besonders bevorzugt enthält dieser heterogene Kupfer-haltige Katalysator, bezogen auf das Gesamtgewicht des Katalysators
- 2 - 15 Gew.%: Kupfer;
- 2 - 15 Gew.%: Kobalt;
- 0 - 5% Gew.%: mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Vorzugsweise enthält dieser Kupfer-haltige Katalysator, als katalytisch aktive Metalle nur Kupfer und Kobalt, insbesondere jeweils (aber unabhängig voneinander) 2 bis 15 Gew.%, besonders bevorzugt 3 bis 10 Gew.%, insbesondere bevorzugt 3 bis 8 Gew. %, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Katalysator, bezogen auf das Gesamtgewicht des Katalysators
- 5 - 40 Gew.%: Kupfer;
- 20 - 80 Gew.%: Kobalt;
- 0 - 10% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur.

Besonders bevorzugt enthält dieser heterogene Kupfer-haltige Katalysator, bezogen auf das Gesamtgewicht des Katalysators
- 10 - 25 Gew.%: Kupfer;
- 40 - 70 Gew.%: Kobalt;
- 0,1 - 8% Gew.%: mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur, vorzugsweise Molybdän, Mangan und Phosphor.

Vorzugsweise enthält dieser Kupfer-haltige Katalysator, als katalytisch aktive Metalle bzw. Promotoren nur Kupfer, Kobalt, Molybdän und Mangan, insbesondere 5 bis 40 Gew.% Kupfer, 30 bis 80 Gew.% Kobalt und jeweils unabhängig voneinander 0,1 bis 10 Gew.% Molybdän und Mangan, besonders bevorzugt 10 bis 35 Gew.% Kupfer, 40 bis 75 Gew.% Kobalt und jeweils unabhängig voneinander 0,5 bis 8 Gew.% Molybdän und Mangan, insbesondere bevorzugt 12 bis 25 Gew.% Kupfer, 45 bis 60 Gew.% Kobalt und jeweils unabhängig voneinander 0,5 bis 7 Gew.% Molybdän und Mangan bezogen auf das Gesamtgewicht des Katalysators.

Der Katalysator weist üblicherweise eine BET-Oberfläche (bestimmt nach DIN 66131) von 50 bis zu 150 m²/g, vorzugsweise von 70 bis 130 m²/g, insbesondere von 75 bis 120 m²/g auf. In der Regel liegt das Porenvolumen des Katalysators (bestimmt mittel Hg-Porosimetrie nach DIN 66133) bei 0,1 bis 0,4 ml/g, vorzugsweise bei 0,15 bis 0,35 ml/g, insbesondere bei 0,15 bis 0,3 ml/g.

Die Herstellung des Katalysators kann aber auch nach üblichen Verfahren erfolgen (A. Farkas, in Ullmann's Encyclopedia of Industrial Chemistry, Electronic Release 2000, Kapitel 5,3, 5,4, 5,6 bis 5,10).

Beispielsweise ist es möglich den Träger aus entsprechenden Verbindungen herzustellen, die sich beim Calcinieren in das Oxid des jeweiligen Trägers umwandeln. Hierfür sind insbesondere Hydroxide, Carbonate und Carboxylate geeignet. Das Oxid bzw. der entsprechende Precursor, der beim Calcinieren in das Oxid des jeweiligen Trägers umgewandelt wird, kann nach an sich bekannten Verfahren, wie z.B. nach dem Sol-Gel-Verfahren, durch Fällung, Entwässern der entsprechenden Carboxylate, Trockenmischen, Aufschlämmen oder Sprühtrocknen, hergestellt werden. Bei der Fällung werden üblicherweise lösliche Salze von Aluminium, Titan, Zirconium etc. eingesetzt, wie z.B. die entsprechenden Halogenide, vorzugsweise Chlorid, Alkoxide, Nitrat etc., vorzugsweise Nitrat von Aluminium, Titan, Zirconium etc. Weiterhin ist es möglich Stabilisatoren in den Träger nach üblichen Verfahren einzubauen. Ebenso können Hilfsmittel in den Träger eingearbeitet werden, welche die Formgebung des Trägers erleichtern, wie z.B. Graphit oder Stearinsäure. Anschließend erfolgt die Formgebung. In der Regel werden Stränge, Tabletten, Kugeln, Splitt, Monolithe etc., nach den üblichen Verfahren hergestellt.

Die Calcinierung erfolgt üblicherweise mit Luft oder einem Gemisch aus Luft und Stickstoff, bei einer Temperatur von 300 bis 800°C, bevorzugt bei 500 bis 600°C. Es kann von Vorteil sein der Luft bzw. dem Luft/Stickstoff-Gemisch Wasserdampf beizumengen.

Auf den Träger können nun die erfindungsgemäßen katalytisch aktiven Metalle bzw. Promotoren aufgebracht werden. Üblicherweise wird der Träger mit einer Lösung eines entsprechenden Metall-Precursors bzw. Promoter-Precursor imprägniert oder in dierser getränkt. Die Imprägnierung kann nach der incipient-wetness Methode erfolgen, wobei das poröse Volumen des Trägers durch in etwa gleiches Volumen an Imprägnierlösung aufgefüllt wird und man - ggf. nach einer Reifung - den Träger trocknet; oder man arbeitet mit einem Überschuss an Lösung, wobei das Volumen dieser Lösung größer ist als das poröse Volumen des Trägers. Hierbei wird der Träger mit in der Imprägnierlösung gemischt und ausreichend lange gerührt. Die überschüssige Imprägnierlösung wird abgeschüttelt, abzentrifugiert oder durch Filtration abgetrennt. Von Fall zu Fall kann auch der Zusatz von Säuren, Neutralsalzen oder Basen die Imprägnierung/Tränkung erleichtern. Eine durchgehende Imprägnierung des Trägers kann von Fall zu Fall erreicht werden, indem z.B. die Lösung während des Imprägnierens/Tränkens erwärmt wird, oberflächenaktive Substanzen zugegeben werden oder der Träger evakuiert wird. Weiterhin ist es möglich den Träger mit einer Lösung des entsprechenden Precursors zu besprühen. Hierbei wird der entsprechende Träger mit einer Lösung des entsprechenden Metall-Precursors bzw. Promoter-Precursor, welche so bemessen ist, dass der Träger die Lösung aufnimmt, behandelt.

Aber auch andere dem Fachmann bekannte Herstellmethoden, wie z.B. Chemical Vapor Deposition, Soltränkung etc. sind möglich.

Geeignete Metall-Precursoren sind entsprechende lösliche Metallsalze, u.a. Halogenide, insbesondere Chlorid, Nitrat, Acetat, alkalische Carbonate, Formiat, Oxalat, Citrat, Tartrat.

Die Aufbringung der Metall- bzw. Promotor-Precursoren nach den voranstehend genannten Methoden kann gemeinsam oder nacheinander erfolgen. Es kann auch von Vorteil sein, hierbei eine gewisse Reihenfolge einzuhalten.

Aber auch andere dem Fachmann bekannte Herstellmethoden, wie z.B. Chemical Vapor Deposition, Soltränkung etc. sind möglich.

Der Träger, auf dem die erfindungsgemäßen katalytisch aktiven Metall-Precursoren, aufgetragen wird, wird nun calciniert. Die Calcinierung erfolgt üblicherweise mit Luft oder einem Gemisch aus Luft und Stickstoff, bei einer Temperatur von 300 bis 800°C, bevorzugt bei 400 bis 600 °C. Es kann von Vorteil sein der Luft bzw. dem Luft/Stickstoff-Gemisch Wasserdampf beizumengen.

Nach der Calcinierung wird der heterogene Kupfer-haltige Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsaure vermischt, mittels einer Tablettenpresse zu Formlingen verpreßt und tempert (warmebehandelt). Die Tempertemperaturen entsprechen dabei im allgemeinen den Temperaturen bei der Calcinierung.

Es können aber auch zur Herstellung der heterogenen Kupfer-haltigen Katalysatoren Fällmethoden angewendet werden. So können diese beispielsweise durch eine gemeinsame Fällung der Metall-/bzw. Promotorprecursoren aus einer diese Metalle/Promotoren enthaltenden, wässrigen Salzlösung mittels Mineralbasen in Gegenwart einer Aufschlämmung einer schwerlöslichen, sauerstoffhaltigen Trägerprecursorverbindung oder des Trägers selbst und anschließendes Waschen, Trocknen und Calcinieren des erhaltenen Prazipitats erhalten werden.

Als schwerlösliche, sauerstoffhaltige Trägerprecursorverbindungen bzw. Träger selbst können beispielsweise Oxide, Oxihydrate, Phosphate, Borate und Silikate verwendet werden, beispielsweise Oxide, Oxihydrate, Phosphate, Borate und Silicate, wie z.B. Zirkoniumdioxid, Zirkoniumoxidhydrat, Zirkoniumphosphate, -borate und -silikate, Siliciumdioxid, Aluminiumoxid, Aluminiumoxihydrat, Titandioxid und dem Fachmann weitere bekannte Verbindungen. Die Aufschlammungen der schwerlöslichen Trägerprecursorverbindungen bzw. Träger selbst können durch Suspendieren feinkörniger Pulver dieser Trägerprecursorverbindungen bzw. Träger selbst in Wasser unter kräftigem Rühren hergestellt werden. Vorteilhaft werden diese Aufschlämmungen durch Ausfällen der schwerlöslichen Trägerprecursorverbindungen aus wässrigen Salzlösungen mittels Mineralbasen erhalten.

Insbesondere werden die erfindungsgemäßen heterogenen Kupfer-haltigen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Warme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollstandig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fallungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle/Promotoren. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fallungsverfahren erhaltenen Niederschläge werden wie üblich zu den erfindungsgemäßen heterogenen Kupfer-haltigen Katalysatoren weiterverarbeitet. Nach dem Waschen werden sie im Allgemeinen bei 80 bis 200 °C, vorzugsweise bei 100 bis 150 °C, getrocknet und danach calciniert. Die Calcinierung (Wärmebehandlung) wird im Allgemeinen bei Temperaturen zwischen 300 und 800 °C, vorzugsweise bei 400 bis 600 °C, insbesondere bei 450 bis 550 °C ausgeführt.

Nach der Calcinierung wird der heterogene Kupfer-haltige Katalysator zweckmäßigerweise konditioniert, sei es, dass man ihn durch Vermahlen auf eine bestimmte Korngröße einstellt oder dass man ihn nach seiner Vermahlung mit Formhilfsmittelnwie Graphit oder Stearinsaure vermischt, mittels einer Tablettenpresse zu Formlingen verpresst und tempert (wärmebehandelt). Die Tempertemperaturen entsprechen dabei im Allgemeinen den Temperaturen bei der Calcinierung.

Die auf diese Weise hergestellten heterogen Kupfer-haltigen Katalysatoren enthalten die katalytisch aktiven Metalle/Promotoren in Form eines Gemisches ihrer sauerstoffhaltigen Verbindungen, d.h. insbesondere als Oxide und Mischoxide.

Die auf diese Weise hergestellten heterogen Kupfer-haltigen Katalysatoren können als solche gelagert werden.

Der so erhaltene Katalysator kann vor seinem Einsatz, in der diaselektiven Hydrierung von Verbindungen der Formel I, aktiviert werden. Hierzu wird er mit Wasserstoff oder einem Gemisch aus Wasserstoff und Stickstoff bei Temperaturen von 100 bis 300°C, behandelt. Hierbei kann es von Vorteil sein, mit einem niedrigen Wasserstoffanteil in dem Wasserstoff/Stickstoff-Gemisch zu beginnen und den Wasserstoffanteil kontinuierlich oder stufenweise im Verlauf des Aktivierungsprozesses zu erhöhen. Die Vorreduktion kann z.B. zunächst bei 150 bis 200°C über einen Zeitraum von 12 bis 20 Stunden in einer Stickstoff/Wasserstoffatmosphäre durchgeführt werden und anschließend noch bis ca. 24 Stunden bei 200 bis 300°C in einer Wasserstoffatmosphäre weitergeführt werden.

Die Aktivierung des Katalysators wird in der Regel in dem Reaktor, in der die erfindungsgemäße Hydrierung erfolgen soll, durchgeführt. Es ist aber auch möglich die Aktivierung des Katalysators vor dem Einbau in den entsprechenden Reaktor vorzunehmen.

Üblicherweise wird der Katalysator in reduzierter Form in die erfindungsgemäße Hydrierung eingesetzt. Hierbei kann es von Vorteil sein, den in reduzierter Form vorliegenden Katalysator nochmals zu aktivieren. Hierzu wird er mit Wasserstoff oder einem Gemisch aus Wasserstoff und einem Inertgas, wie z.B. Stickstoff, bei Temperaturen von Raumtemperatur bis 100 °C, vorzugsweise bei 150 bis 300 °C, und einem Wasserstoffdruck von 10 bis 60 bar, vorzugsweise bei max. 50 bar, behandelt. Hierbei kann es von Vorteil sein, mit Wasserstoff ohne Inertgas zu aktivieren. Es kann aber auch von Vorteil sein mit einem Gemisch von Wasserstoff und Inertgas zu aktivieren, wobei mit dem Wasserstoff/Inertgas-Gemisch zu beginnen und den Wasserstoffanteil kontinuierlich im Verlauf des Aktivierungsprozesses zu erhöhen.

Es ist aber auch möglich den Katalysator, in seiner oxidischen Form oder auch in seiner reduzierten Form, ohne weitere vorangehende Aktivierung in die diaselektiven Hydrierung von Iminen der Formel I einzusetzen.

### Zur Verfahrensdurchführung

Die Umsetzung erfolgt in der Regel in einem Lösungsmittel. Es ist aber auch möglich die Umsetzung in Substanz durchzuführen, insbesondere wenn das Imin der Formel I bei der Reaktionstemperatur flüssig ist. Als Lösungsmittel werden unter den Reaktionsbedingungen inerte Lösungsmittel, wie Alkohole, beispielseweise Methanol, Ethanol, n-Propanol, Isopropanol, Cyclopentanol, Cyclohexanol, Ethylenglycol, Propylenglycol etc., aromatische Kohlenwasserstoffe, beispielsweise Benzol, Toluol, Ethylbenzol, Xylol etc.,Ether, beispielsweise Diethylether, Methyl-tert.-butylether, Ethylengylcoldimethylether, Tetrahydrofuran, Dioxan, dipolar aprotische Lösungsmittel, beispielsweise N-Methylpyrrolidon, Dimethylsulfoxid, Sulfolan, Dimethylformamid, Dimethylacetamid etc., oder Gemische hiervon eingesetzt. Vorzugsweise wird die Umsetzung in einem Alkohol, wie Methanol, Ethanol, n-Propanol, Isopropanol,Cyclopentanol, Cyclohexanol, Ethylenglycol, Propylenglycol etc., vorzugsweise Methanol, Ethanol und Isopropanol oder einen aromatischen Kohlenwasserstoff, wie Benzol, Toluol, Ethylbenzol, Xylol etc., vorzugsweise Toluol oder Ethylbenzol, oder Gemischen hiervon durchgeführt.

Üblicherweise wird die Umsetzung bei einer Temperatur von Raumtemperatur bis Rückflusstemperatur des Reaktionsgemisches, in der Regel von Raumtemperatur bis 200°C, vorzugsweise von 50°C bis 150°C durchgeführt.

Die Umsetzung wird in Gegenwart von Wasserstoff durchgeführt. Die Umsetzung kann in Gegenwart von reinem Wasserstoff oder auch in Gegenwart eines Gasgemisch, welches Wasserstoff enthält, durchgeführt werden.

Der Wasserstoff bzw. der Wasserstoff des Wasserstoff-enthaltende Gasstroms kann vollständig oder teilweise zur Reaktion gebracht werden. In letzten Fall kann es von Fall zu Fall von Vorteil sein diesen Gasstrom teilweise oder ganz rückzuführen bzw. im Kreis zu führen. Für den Fall, dass der eingesetzte Kupfer-haltige Kataysator vor der Umsetzung aktiviert wird, dann kann dieser Gasstrom auch hierfür verwendet werden.

Üblicherweise wird der Wasserstoff von technischer Qualität eingesetzt. Der Wasserstoff kann aber auch in der Form eines Wasserstoff enthaltenden Gases, d.h. als Beimengung eines Inertgases, wie Stickstoff, Helium, Neon, Argon oder Kohlendioxid, vorzugsweise Stickstoff oder Argon, eingesetzt werden.

Im Allgemeinen wird die Reaktion bei einem Druck von 1 bis 200 bar, vorzugsweise von 40 bis 150 bar, insbesondere von 50 bis 100 bar, durchgeführt. Es ist möglich den Druck stufenweise bis zum gewünschten Druck zu erhöhen oder auch kontinuierlich.

Das erfindungsgemäße Verfahren kann diskontinuierlich, semikontinuierlich oder kontinuierlich durchgeführt werden.

Bei einer diskontinuierlichen Verfahrensweise wird das Rektionsgemisch nach üblichen Verfahren aufgearbeitet, indem man z.B. den Katalysator abtrennt, beispielsweise durch Filtration, Absetzen lassen und Abtrennen der flüssigen Phase oder durch Zentrifugation, und von dem so erhaltenen Filtrat, Überstand bzw. Zentrifugat wird das Lösungsmittel, z.B. durch abdestillieren, entfernt. Von Fall zu Fall kann es von Vorteil sein ein Filtrationshilfmittel, wie z.B. Celite, zu verwenden, falls der Katalysator abfiltriert wird.

Die Umsetzung kann in der Flüssigphase, beispielsweise in einem Rührautoklaven, einer Blasensäule, einem Umlaufreaktor, wie etwa einem Schlaufenreaktor oder einem Festbettreaktor, erfolgen.

Ein besonderer Vorteil des erfindungsgemäßen Verfahrens ist die Recyclierbarkeit des heterogenen Iminierungskatalysators. Der Katalysator kann nach der Umsetzung in einfacher Weise, z. B. durch Filtration abgetrennt und wieder eingesetzt werden.

Die Reaktionsdauer wird vorzugsweise so gewählt, dass der Umsatz einer der Ausgangskomponenten nahezu vollständig ist. Es kann aber auch früher abgebrochen werden. Typischer Weise wird die Reaktion zwischen 0,1-200 Stunden, bevorzugt zischen 0,1 -180 Stunden gefahren.

Die Reaktion wird vorzugsweise beendet, wenn die im Unterschuss eingesetzte Komponente vollständig umgesetzt wurde. Es kann aber auch von Vorteil sein, die Reaktion vor dem Erreichen des Vollumsatzes zu beenden.

Eine Aufarbeitung des Produktgemisches kann in üblicher und dem Fachmann bekannter Weise erfolgen, das Produkt kann z. B. durch Destillation, Extraktion oder Kristallisation erhalten werden.

Bevorzugte Produkte des erfindungsgemäßen Verfahrens sind die Produkte der Umsetzung von (R)- bzw. (S)-(1-Phenyl-ethyl)amin mit Ethyl-methyl-Keton, Methylisopropyl-keton oder Acetophenon.

Das erfindungsgemäße Verfahren ist ein besonders einfaches und kostengünstiges verfahren zur Herstellung von Verbindungen der Formel I. Die Anzahl der Verfahrensstufen kann gegenüber dem Stand der Technik vermindert werden. Die Reaktionsdurchführung und die Aufarbeitung sind einfach. Die Produkte haben eine hohe Diastereoselektivität, erkennbar in den Beispielen an einem hohen Wert für das Verhältnis der Diastereoisomeren RR/ RS bzw. SS/SR.

### Weitere Umsetzung

Die Amine der Formel I können hydrogenolytisch zu den chiralen Aminen der Formel VIII, 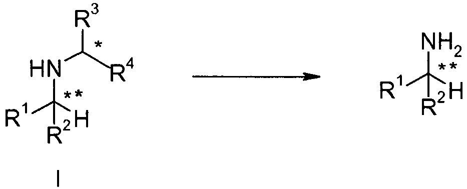 wobei die Reste R¹ und R² die bei den Verbindungen der Formel I gegebene Bedeutung haben, gespalten werden.

Diese Reaktion kann nach üblichen und bekannten Verfahren durchgeführt werden.

Üblicherweise wird diese Hydrogenolyse in einem inerten Lösungsmittel, wie z.B. einem Alkohol, wie Methanol, Ethanol, Iso-Propanol oder Butanol, einem Ether, wie z.B. Tetrahydrofuran, Dioxan, einem Kohlenwasserstoff, wie z.B. Toluol oder Benzol, oder Gemischen hiervon durchgeführt. Die Hydrogenolyse kann mittels Wasserstoff in Gegenwart einer katalytischen Mengeeines Platin-Gruppen-Metallelements, vorzugsweise an Pt/C oder an Pd/C durchgeführt werden. Hierbei wird in der Regel der Wasserstoff im Überschuß eingesetzt. Die Umsetzung erfolgt in der Regel bei Raumtemperatur bis Rückflußtemperatur des Reaktionsgemisches und Normaldruck bis zu einem Druck von 200 bar. Nach Beendigung der Umsetzung wird das Reaktionsgemisch nach dem Fachmann bekannten Methoden aufgearbeitet.

Es ist aber auch möglich die Hydrogenolyse mittels Metallhydride, wie z.B. Lithiumaluminiumhydrid, Natriumboranat, Natriumcycanoborant, Diboran etc. durchzuführen. Hierbei werden die Edukte in der Regel im stöchiometrischen Verhältnis eingesetzt. Von Fall zu Fall kann es auch von Vorteil sein, das Metallhydrid im Überschuß einzusetzten. Die Umsetzung erfolgt in der Regel bei Raumtemperatur bis Rückflußtemperatur des Reaktionsgemisches, bei Normaldruck. Nach Beendigung der Umsetzung wird das Reaktionsgemisch nach dem Fachmann bekannten Methoden aufgearbeitet.

Die Hydrogenolyse der Amine der Formel il kann kontinuierlich, semi-kontinuierlich oder in Batchfahrweise durchgeführt werden.

### Beispiele zur Patentanmeldung

Einsatzstoffe:
Montmorillonit K10: erhältlich von Sigma-Aldrich
Pt/C. 10% Pt auf Kohlenstoffträger
Ni/Cu/Mo analog DE 4428004
Ni/Co/Cu analog DE 19826396

### Messmethoden

Das Diastereomerenverhältnis für Beispiele 1-9 wurde wie folgt bestimmt: Derivatisierung mit Trifluoressigsäure, gaschromatographische Trennung auf Säule BGB 175 Das Diastereomerenverhältnis für Beispiele 10-12 wurde wie folgt bestimmt: Derivatisierung mit Trifluoressigsäure, gaschromatographische Trennung auf Säule Hydrodex beta 6-TBDM

### 1. Umsetzung von 2-Butanon mit (R)- bzw. (S)-(1-Phenyl-ethyl)-amin

### Beispiel 1

Das Keton wird in einem Rundkolben mit (S)-(1-Phenyl-ethyl)-amin, Montmorillonit K10 und Pt/C vorgelegt und für 1 Stunde bei 60 °C gerührt. Danach wird Wasserstoff eingeleitet, bis der Umsatz vollständig ist.
Bei dem Reaktionsprodukt handelt es sich um eine Verbindung der Formel I mit
R¹ = Methyl R² = Ethyl R³ = Methyl R⁴ = Phenyl

**Tabelle 1**

| Bsp. Nr. | Kat1 | Träger | Kat [g] | K10 | Keton [g] | Amin [g] | T [°C] | p [bar] | Laufzeit [h] | Umsatz (Imin) [%] | Verhältnis SS/SR |
|---|---|---|---|---|---|---|---|---|---|---|---|
| 1 | Pt (10) | C | 2 | 1,5 | 216 | 73 | 60 | ND | 4,5 | >99 | 66/34 |

### Beispiele 2-9

Das Keton wird mit dem (R)-(1-Phenyl-ethyl)-amin (Tabelle 2) bzw. (S)-(1-Phenyl-ethyl)-amin (Tabelle 3) und den beiden Katalysatoren (Montmorillonit K10 für die Iminierung und einen Edelmetallkatalysator "Kat" für die Hydrierung) im Autoklaven vorgelegt. Die Apparatur wir mit Stickstoff inertisiert und die Mischung bei der angegebenen Temperatur für eine Stunde gerührt. Dazu wird Wasserstoff bis zu dem gewünschten Druck zugepresst und mehrere Stunden hydriert (Laufzeit). Nach Beendigung wird der Autoklav entspannt.
Bei dem Reaktionsprodukt handelt es sich um eine Verbindung der Formel I mit
R¹ = Methyl R² = Ethyl R³ = Methyl R⁴ = Phenyl

**Tabelle 2**

| Bsp Nr. | Kat 1 | Träger | Kat [g] | K10 | Keton [g] | Amin [g] | Autoklavengröße [ml] | T[°C] | p [bar] | Laufzeit [h] | Umsatz (Imin) [%] | Verhältnis RR/RS |
|---|---|---|---|---|---|---|---|---|---|---|---|---|
| 2 | Ni/Cu/Mo (40/13/1) | ZrO₂ | 4 | 0,6 | 58 | 49 | 300 | 100 | 200 | 24 | >99 | 86/14 |
| 3 | Ni/Cu/Mo (40/13/1) | ZrO₂ | 1,7 | 0,5 | 50 | 42 | 300 | 100 | 70 | 24 | >99 | 86/14 |
| 4 | Ni/Co/Cu (21/21/11) | ZrO₂ | 1,7 | 0,5 | 50 | 42 | 300 | 100 | 70 | 24 | >99 | 85/15 |
| 5 | Pt (10) | C | 4 | 0,6 | 58 | 49 | 300 | 100 | 200 | 24 | >99 | 74/26 |

**Tabelle 3**

| Bsp. Nr. | Kat 1 | Träger | Kat [g] | K10 | Keton [g] | Amin [g] | Autoklavengröße [ml] | T[°C] | p [bar] | Laufzeit [h] | Umsatz (Imin) [%] | Verhältnis SS/SR |
|---|---|---|---|---|---|---|---|---|---|---|---|---|
| 6 | Ni/Cu/Mo (40/13/1) | ZrO₂ | 4 | 0,6 | 58 | 49 | 300 | 100 | 200 | 24 | >99 | 92/8 |
| 7 | Pt (10) | C | 4 | 0,6 | 58 | 49 | 300 | 100 | 200 | 24 | >99 | 74/26 |
| 8 | Ni/Cu/Mo (40/13/1) | ZrO₂ | 5 | 1 | 58 | 49 | 300 | 100 | 100 | 24 | 98 | 92/8 |
| 9 | Pt (10) | C | 1,9 | 0,6 | 58 | 49 | 300 | RT | 50 | 24 | >99 | 63/37 |

### 2. Umsetzung von 3-Methyl-2-butanon mit (R)- bzw. (S)-(1-Phenyl-ethyl)-amin

### Beispiele 10-11

Das Keton wird in einem Rundkolben mit (R)-(1-Phenyl-ethyl)-amin (Tabelle 4) bzw. (S)-(1-Phenyl-ethyl)-amin (Tabelle 5), Montmorillonit und Pt/C vorgelegt und für 1 Stunde bei 60 °C gerührt. Danach wird Wasserstoff eingeleitet, bis der Umsatz vollständig ist.
Bei dem Reaktionsprodukt handelt es sich um eine Verbindung der Formel I mit
R¹ = Methyl R² = iso-Propyl R³ = Methyl R⁴ = Phenyl

**Tabelle 4**

| Bsp. Nr. | Kat 1 | Träger | Kat [g] | K10 | Keton [g] | Amin [g] | T [°C] | p [bar] | Laufzeit [h] | Umsatz (Imin) [%] | Verhältnis RR/RS |
|---|---|---|---|---|---|---|---|---|---|---|---|
| 10 | Pt (10) | C | 8 | 4 | 855 | 601 | 60 | ND | 14 | >99 | 84/16 |

**Tabelle 5**

| Bsp. Nr. | Kat 1 | Träger | Kat [g] | K10 | Keton [g] | Amin [g] | T [°C] | p [bar] | Laufzeit [h] | Umsatz (Imin) [%] | Verhältnis SS/SR |
|---|---|---|---|---|---|---|---|---|---|---|---|
| 11 | Pt (10) | C | 4,8 | 1,5 | 172 | 121 | 60 | ND | 6 | >99 | 92/8 |

### Beispiele 12-18

Das Keton wird mit dem (R)-(1-Phenyl-ethyl)-amin (Tabelle 6) bzw. (S)-(1-Phenyl-ethyl)-amin (Tabelle 7) und den beiden Katalysatoren (Montmorillonit K10 für die Iminierung und einen Edelmetallkatalysator "Kat" für die Hydrierung) im Autoklaven vorgelegt. Die Apparatur wir mit Stickstoff inertisiert und die Mischung bei der angegebenen Temperatur für eine Stunde gerührt. Dazu wird Wasserstoff bis zu dem gewünschten Druck zugepresst und mehrere Stunden hydriert (Laufzeit). Nach Beendigung wird der Autoklav entspannt.
Bei dem Reaktionsprodukt handelt es sich um eine Verbindung der Formel I mit
R¹ = Methyl R² = iso-Propyl R³ = Methyl R⁴ = Phenyl

**Tabelle 6**

| Bsp. Nr. | Kat 1 | Träger | Kat [g] | K10 | Keton [g] | Amin [g] | Autoklavengröße [ml] | T [°C] | p [bar] | Laufzeit [h] | Umsatz (Imin) [%] | Verhältnis RR/RS |
|---|---|---|---|---|---|---|---|---|---|---|---|---|
| 12 | Pt (10) | C | 2 | 0,6 | 50 | 35,1 | 300 | 100 | 100 | 24 | >99 | 72/28 |
| 13 | Pt (10) | C | 2 | 6 | 50 | 35,1 | 300 | 100 | 100 | 24 | >99 | 70/30 |
| 14 | Ni/Cu/Mo (40/13/1) | ZrO₂ | 1,4 | 0,5 | 50 | 35,2 | 300 | 100 | 70 | 24 | >99 | 96/4 |
| 15 | Ni/Co/Cu (21/21/11) | ZrO₂ 1_{'}4 ZrO₂ | 1,4 | 0,5 | 50 | 35,2 | 300 | 100 | 70 | 24 | >199 | 98/2 |

**Tabelle 7**

| Bsp. Nr. | Kat 1 | Träger | Kat [g] | K10 | Keton [g] | Amin [g] | Autoklavengröße [ml] | T[°C] | p [bar] | Laufzeit [h] | Umsatz (Imin) [%] | Verhältnis SS/SR |
|---|---|---|---|---|---|---|---|---|---|---|---|---|
| 16 | Pt (10) | C | 1,9 | 0,6 | 69 | 49 | 300 | 60 | 50 | 24 | >99 | 83/17 |
| 17 | Ni/Cu/Mo (40/13/1) | ZrO₂ | 5 | 1 | 69 | 49 | 300 | 150 | 200 | 24 | >99 | 96/4 |
| 18 | Pt (10) | C | 1,9 | 0,6 | 69 | 49 | 300 | 60 | 50 | 24 | >99 | 82/18 |

## Patentansprüche

1. Verfahren zur Herstellung von Aminen der Formel I, 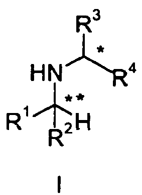 durch Umsetzung einer Carbonylverbindung der Formel II mit einem Amin der Formel III 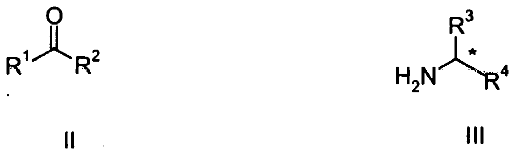 wobei
R¹ und R² voneinander verschieden sind und jeweils für einen organischen Rest mit 1 bis 20 C-Atomen, der gegebenenfalls auch Heteroatome enthalten kann, stehen,
R³ für eine C₁-C₆-Alkylgruppe steht und
R⁴ für eine Arylgruppe steht, welche partiell oder vollständig halogeniert sein kann und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;
und
* für die S oder R-Konfiguration, und
** für die S und/oder R-Konfiguration;
stehen;
**dadurch gekennzeichnet, dass** man die Umsetzung in Gegenwart eines heterogen Iminierungs-Katalysators, eines Hydrierkatalysators und Wasserstoff durchführt, wobei es sich bei dem heterogenen Iminierungs-Katalysator um anorganische Oxide oder Mischoxide handelt.

2. Verfahren gemäß Anspruch 1, **dadurch gekennzeichnet, dass** R1 und R2 voneinander verschieden sind und jeweils für einen Kohlenwasserstoffrest mit 1 bis 20 C-Atomen stehen.

3. Verfahren gemäß Anspruch 1 oder 2, **dadurch gekennzeichnet, dass** R3 für eine Methylgruppe steht.

4. Verfahren gemäß einem der Ansprüche 1 bis 3, **dadurch gekennzeichnet, dass** R4 für eine Phenyl- oder eine 1-Naphthylgruppe steht.

5. Verfahren gemäß einem der Ansprüche 1 bis 4, **dadurch gekennzeichnet, dass** es sich bei dem heterogenen Iminierungs-Katalysator um Silikate oder Silizium enthaltende Mischoxide handelt.

6. Verfahren gemäß einem der Ansprüche 1 bis 5, **dadurch gekennzeichnet, dass** es sich bei dem Hydrierkatalysator um einen heterogenen, Kupfer-haltigen Katalysator handelt.

7. Verfahren gemäß einem der Ansprüche 1 bis 6, **dadurch gekennzeichnet, dass** der Hydrierkatalysator, bezogen auf sein Gesamtgewicht 1 bis 95 Gew. % Kupfer enthält.

8. Verfahren gemäß einem der Ansprüche 1 bis 7, **dadurch gekennzeichnet, dass** es sich bei dem Kupfer-haltigen Hydrierkatalysator um einen Trägerkatalysator handelt.

9. Verfahren gemäß einem der Ansprüche 1 bis 8, **dadurch gekennzeichnet, dass** die Umsetzung in Gegenwart eines Lösungsmittels oder in Substanz durchgeführt wird.

10. Verfahren gemäß einem der Ansprüche 1 bis 9, **dadurch gekennzeichnet, dass** die Umsetzung bei Normaldruck bis 200 bar durchgeführt wird.

11. Verfahren gemäß einem der Ansprüche 1 bis 10, **dadurch gekennzeichnet, dass** die Umsetzung bei Raumtemperatur bis Rückflusstemperatur des Reaktionsgemisches durchgeführt wird.

12. Verfahren gemäß einem der Ansprüche 1 bis 11, **dadurch gekennzeichnet, dass** die Umsetzung einstufig durchgeführt wird und gegebenenfalls entstehende Zwischenprodukte nicht isoliert werden.

13. Verfahren zur Herstellung von chiralen Aminen der Formel IV, 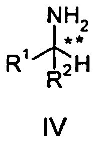 umfassend folgende Schritte
a) Umsetzung einer Carbonylverbindung der Formel II mit einem Amin der Formel III zu einem Armin der Formel I 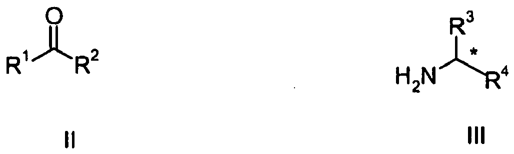 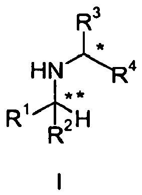 wobei
R¹ und R² voneinander verschieden sind und jeweils für einen organischen Rest mit 1 bis 20 C-Atomen, der gegebenenfalls auch Heteroatome enthalten kann, stehen,
R³ für eine C₁-C₆-Alkylgruppe steht und
R⁴ für eine Arylgruppe steht, welche partiell oder vollständig halogeniert sein kann und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;
und
* für die S oder R-Konfiguration, und
* * für die S und/oder R-Konfiguration; stehen;
b) und anschließende hydrogenolytische Spaltung des aus Schritt a) erhaltenen Amins der Formel I,
**dadurch gekennzeichnet, dass** man die Umsetzung von Schritt a) in Gegenwart eines heterogen Iminierungs-Katalysators, eines Hydrierkatalysators und Wasserstoff durchführt, wobei es sich bei dem heterogenen Iminierungs-Katalysator um anorganische Oxide oder Mischoxide handelt.

## Claims

1. A process for preparing amines of the formula I, 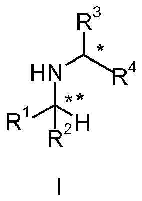 by reacting a carbonyl compound of the formula II with an amine of the formula III 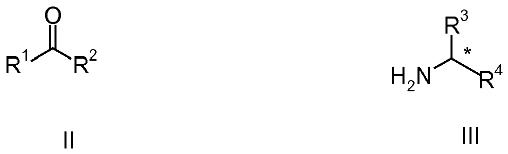 where
R¹ and R² are different and are each an organic radical which has from 1 to 20 carbon atoms and may optionally also comprise heteroatoms,
R³ is a C₁-C₆-alkyl group and
R⁴ is an aryl group which may be partly or fully halogenated and/or may bear from one to three radicals from the group of cyano, nitro, C₁-C₆-alkyl, C₁-C₆-haloalkyl, hydroxyl, C₁-C₆-hydroxyalkyl, C₁-C₆-alkoxy, C₁-C₆-haloalkoxy, hydroxycarbonyl, C₁-C₆-alkoxycarbonyl, C₁-C₆-alkylamino, di(C₁-C₆-alkyl)amino, aryl and aryl(C₁-C₆-alkyl);
and
* represents the S or R configuration, and
** represents the S and/or R configuration;
which comprises performing the reaction in the presence of a heterogeneous imination catalyst, of a hydrogenation catalyst and of hydrogen, the heterogeneous imination catalyst comprising inorganic oxides or mixed oxides.

2. The process according to claim 1, wherein R1 and R2 are different and are each a hydrocarbon radical having from 1 to 20 carbon atoms.

3. The process according to claim 1 or 2, wherein R3 is a methyl group.

4. The process according to any one of claims 1 to 3, wherein R4 is a phenyl or a 1-naphthyl group.

5. The process according to any one of claims 1 to 4, wherein the heterogeneous imination catalyst comprises silicates or mixed oxides comprising silicon.

6. The process according to any one of claims 1 to 5, wherein the hydrogenation catalyst is a heterogeneous copper catalyst.

7. The process according to any one of claims 1 to 6, wherein the hydrogenation catalyst, based on its total weight, comprises from 1 to 95% by weight of copper.

8. The process according to any one of claims 1 to 7, wherein the copper hydrogenation catalyst is a supported catalyst.

9. The process according to any one of claims 1 to 8, wherein the reaction is performed in the presence of a solvent or in bulk.

10. The process according to any one of claims 1 to 9, wherein the reaction is performed at from standard pressure to 200 bar.

11. The process according to any one of claims 1 to 10, wherein the reaction is performed at from room temperature to reflux temperature of the reaction mixture.

12. The process according to any one of claims 1 to 11, wherein the reaction is performed in one stage and any intermediates formed are not isolated.

13. A process for preparing chiral amines of the formula IV, 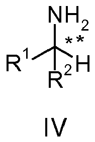 comprising the following steps
a) reacting a carbonyl compound of the formula II with an amine of the formula III to give an amine of the formula I 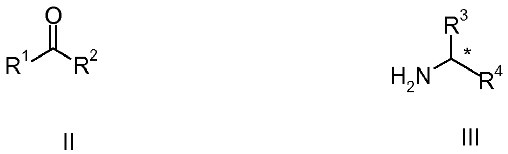 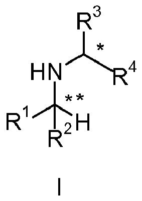 where
R¹ and R² are different and are each an organic radical which has from 1 to 20 carbon atoms and may optionally also comprise heteroatoms,
R³ is a C₁-C₆-alkyl group and
R⁴ is an aryl group which may be partly or fully halogenated and/or may bear from one to three radicals from the group of cyano, nitro, C₁-C₆-alkyl, C₁-C₆-haloalkyl, hydroxyl, C₁-C₆-hydroxyalkyl, C₁-C₆-alkoxy, C₁-C₆-haloalkoxy, hydroxycarbonyl, C₁-C₆-alkoxycarbonyl, C₁-C₆-alkylamino, di(C₁-C₆-alkyl)amino, aryl and aryl(C₁-C₆-alkyl);
and
* represents the S or R configuration, and
** represents the S and/or R configuration;
b) and subsequently hydrogenolytically cleaving the amine of the formula I obtained from step a),
which comprises performing the reaction in step a in the presence of a heterogeneous imination catalyst, of a hydrogenation catalyst and of hydrogen, the heterogeneous imination catalyst comprising inorganic oxides or mixed oxides.

## Revendications

1. Procédé de fabrication d'amines de formule I 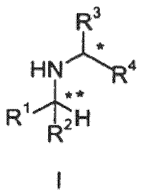 par mise en réaction d'un composé de carbonyle de formule II avec une amine de formule III 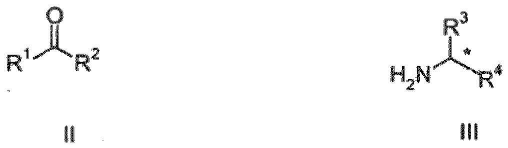 dans lesquelles
R¹ et R² sont différents l'un de l'autre et représentent chacun un radical organique de 1 à 20 atomes C, qui peut éventuellement également contenir des hétéroatomes,
R³ représente un groupe alkyle en C₁-C₆, et
R⁴ représente un groupe aryle, qui peut être halogéné en partie ou en totalité et/ou qui peut porter un à trois radicaux du groupe constitué par cyano, nitro, alkyle en C₁-C₆, halogénoalkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, alkylamino en C₁-C₆, di(alkyle en C₁-C₆) amino, aryle et aryl(alkyle en C₁-C₆) ;
et
* représente la configuration S ou R, et
** représente la configuration S et/ou R ;
**caractérisé en ce que** la réaction est réalisée en présence d'un catalyseur d'imination hétérogène, d'un catalyseur d'hydrogénation et d'hydrogène, le catalyseur d'imination hétérogène consistant en des oxydes ou oxydes mixtes inorganiques.

2. Procédé selon la revendication 1, **caractérisé en ce que** R¹ et R² sont différents l'un de l'autre et représentent chacun un radical hydrocarboné de 1 à 20 atomes C.

3. Procédé selon la revendication 1 ou 2, **caractérisé en ce que** R³ représente un groupe méthyle.

4. Procédé selon l'une quelconque des revendications 1 à 3, **caractérisé en ce que** R⁴ représente un groupe phényle ou 1-naphtyle.

5. Procédé selon l'une quelconque des revendications 1 à 4, **caractérisé en ce que** le catalyseur d'imination hétérogène consiste en des oxydes mixtes contenant des silicates ou du silicium.

6. Procédé selon l'une quelconque des revendications 1 à 5, **caractérisé en ce que** le catalyseur d'hydrogénation consiste en un catalyseur hétérogène contenant du cuivre.

7. Procédé selon l'une quelconque des revendications 1 à 6, **caractérisé en ce que** le catalyseur d'hydrogénation contient, par rapport à son poids total, 1 à 95 % en poids de cuivre.

8. Procédé selon l'une quelconque des revendications 1 à 7, **caractérisé en ce que** le catalyseur d'hydrogénation contenant du cuivre consiste en un catalyseur supporté.

9. Procédé selon l'une quelconque des revendications 1 à 8, **caractérisé en ce que** la réaction est réalisée en présence d'un solvant ou en substance.

10. Procédé selon l'une quelconque des revendications 1 à 9, **caractérisé en ce que** la réaction est réalisée à une pression allant de la pression normale à 200 bar.

11. Procédé selon l'une quelconque des revendications 1 à 10, **caractérisé en ce que** la réaction est réalisée à une température allant de la température ambiante à la température de reflux du mélange réactionnel.

12. Procédé selon l'une quelconque des revendications 1 à 11, **caractérisé en ce que** la réaction est réalisée en une étape et les produits intermédiaires éventuellement formés ne sont pas isolés.

13. Procédé de fabrication d'amines chirales de formule IV 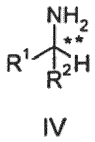 comprenant les étapes suivantes :
a) la mise en réaction d'un composé de carbonyle de formule II avec une amine de formule III pour former une amine de formule I 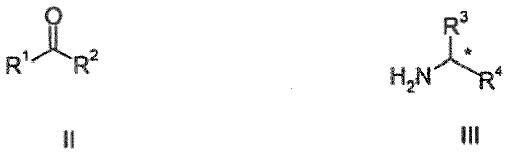 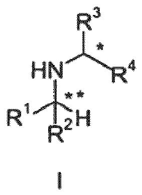 dans lesquelles
R¹ et R² sont différents l'un de l'autre et représentent chacun un radical organique de 1 à 20 atomes C, qui peut éventuellement également contenir des hétéroatomes,
R³ représente un groupe alkyle en C₁-C₆, et
R⁴ représente un groupe aryle, qui peut être halogéné en partie ou en totalité et/ou qui peut porter un à trois radicaux du groupe constitué par cyano, nitro, alkyle en C₁-C₆, halogénoalkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, alkylamino en C₁-C₆, di(alkyle en C₁-C₆) amino, aryle et aryl(alkyle en C₁-C₆) ;
et
* représente la configuration S ou R, et
** représente la configuration S et/ou R ;
b) puis le clivage hydrogénolytique de l'amine de formule I obtenue à l'étape a),
**caractérisé en ce que** la réaction de l'étape a) est réalisée en présence d'un catalyseur d'imination hétérogène, d'un catalyseur d'hydrogénation et d'hydrogène, le catalyseur d'imination hétérogène consistant en des oxydes ou oxydes mixtes inorganiques.
